# EUROPEAN PATENT APPLICATION

(11) **EP 4 464 332 A1**
(43) Date of publication of application: **20.11.2024**
(21) Application number: 24182166.9
(22) Date of filing: 31.03.2015
(51) Int. Cl.: A61K 38/26, C07K 19/00, C07K 1/02, A61K 38/29, C07K 16/46, A61P 3/10, A61K 38/28

(54) **COMPOSITION FOR IMPROVING THE SOLUBILITY OF A PROTEIN OR PEPTIDE BY USING IMMUNOGLOBULIN FC FRAGMENT LINKAGE**

(30) Priority: 31.03.2014 KR 20140038032
(62) Divisional of application: 21169291.8
(71) Applicant: Hanmi Pharm. Co., Ltd., Hwaseong-si, Gyeonggi-do 445-958 (KR)
(72) Inventor: LIM, Hyung Kyu, 445-320 Gyeonggi-do (KR); LEE, Jong Soo, 463-725 Gyeonggi-do (KR); KIM, Dae Jin, 445-764 Gyeonggi-do (KR); BAE, Sung Min, 463-901 Gyeonggi-do (KR); JUNG, Sung Youb, 448-130 Gyeonggi-do (KR); KWON, Se Chang, 135-506 Seoul (KR)
(74) Representative: Mewburn Ellis LLP

(57) **Abstract**

The present invention relates to a method for improving the solubility of a physiologically active protein or peptide compared to that of a physiologically active protein or peptide which is not conjugated to an immunoglobulin Fc fragment, in which the method comprises conjugating the physiologically active protein or peptide to an immunoglobulin Fc fragment; and a composition for improving the solubility of a physiologically active protein or peptide, comprising an immunoglobulin Fc fragment, in which the composition improves the solubility compared to a composition without an immunoglobulin Fc fragment..

## Description

### [Technical Field]

The present invention relates to a method for improving the solubility of a physiologically active protein or peptide compared to that of a physiologically active protein or peptide which is not conjugated to an immunoglobulin Fc fragment, the method including the step of conjugating the physiologically active protein or peptide to the immunoglobulin Fc fragment, and a composition for improving the solubility of a physiologically active protein or peptide, including an immunoglobulin Fc fragment, in which the composition improves the solubility compared to a composition without an immunoglobulin Fc fragment.

### [Background Art]

Drugs exhibit pharmacological actions only when they are dissolved, and thus solubility of drugs is closely related to efficacy. Water-soluble drugs are easily prepared as injectable formulations, and are dissolved in the gastrointestinal tract when taken as tablets. However, poorly water-soluble drugs exist as aggregates such as pellets in the gastrointestinal tract when taken without solubilization, and they are not dissolved into single molecules and are hardly absorbed. Further, when poorly water-soluble drugs are used as injectable formulations without solubilization, blood vessels are clogged to cause blood clotting. Therefore, poorly water-soluble drugs cannot be used as injectable formulations. Drug solubility is a major challenge in drug formulation.

One of the known drug solubilization methods is to use a surfactant as a solubilizer. Surfactants have both hydrophobic and hydrophilic groups in each molecule. In water, hydrophobic hydrocarbon chains are sequestered inside and hydrophilic groups are exposed to water to form spherical micelles. The drugs can be dissolved by encapsulation in micelles. However, when the surfactant is used at a high concentration in this method, the drugs may not be suitable for intravenous administration. Further, dilution of micro-emulsions below the critical micelle concentration of the surfactants could cause precipitation of the drug in the micelles (Journal of Advanced Pharmacy Education & Research 2(1)32-67 (2012) ISSN 2249-3379). Accordingly, there has been a demand for a method of continuously increasing drug solubility regardless of concentration of the formulation without using surfactants.

### [Disclosure]

### [Technical Problem]

Under this background, the present inventors have made many efforts to develop a method capable of improving the solubility of a drug such as a physiologically active protein or peptide. As a result, they found that when an immunoglobulin Fc fragment is conjugated to the physiologically active protein or peptide, the solubility of the physiologically active protein or peptide is effectively increased to easily prepare a formulation including the physiologically active protein or peptide at a pharmaceutically effective concentration, thereby completing the present invention.

### [Technical Solution]

An object of the present invention is to provide a method for improving the solubility of a physiologically active protein or peptide compared to that of a physiologically active protein or peptide which is not conjugated to an immunoglobulin Fc fragment, wherein the method comprises conjugating the physiologically active protein or peptide to an immunoglobulin Fc fragment.

Another object of the present invention is to provide a composition for improving the solubility of a physiologically active protein or peptide, comprising an immunoglobulin Fc fragment, wherein the composition improves the solubility compared to a composition without an immunoglobulin Fc fragment..

### [Advantageous Effects]

When a physiologically active protein or peptide is conjugated with an immunoglobulin Fc fragment according to the present invention, the solubility of the physiologically active protein or peptide may be effectively improved, and is thereby effective in formulation of various drugs.

### [Description of Drawings]

FIG. 1 shows standard calibration curves of insulin and a long-acting insulin conjugate.
FIG. 2 shows a result of examining solubility of insulin and the long-acting insulin conjugate according to the composition of Table 1, in which insulin shows solubility of 0.628 mg/mL and the long-acting insulin conjugate shows solubility of 15 mg/mL, on the basis of insulin.
FIG. 3 shows standard calibration curves of an oxyntomodulin derivative of SEQ ID NO: 27 and an oxyntomodulin derivative-PEG-immunoglobulin Fc fragment conjugate.
FIG. 4 shows a result of examining solubility of the oxyntomodulin derivative of SEQ ID NO: 27 and the oxyntomodulin derivative-PEG-immunoglobulin Fc fragment conjugate according to the composition of Table 4, in which the oxyntomodulin derivative shows solubility of 0.188 mg/mL and the conjugate shows solubility of 11 mg/mL, on the basis of oxyntomodulin.

### [Best Mode]

An aspect of the present invention provides a method for improving the solubility of a physiologically active protein or peptide compared to that of a physiologically active protein or peptide which is not conjugated to an immunoglobulin Fc fragment, in which the method includes conjugating the physiologically active protein or peptide to an immunoglobulin Fc fragment.

In a specific embodiment, the immunoglobulin Fc fragment applied to the method according to the present invention is an Fc fragment derived from IgG, IgA, IgD, IgE, or IgM.

In another specific embodiment, the immunoglobulin Fc fragment applied to the method according to the present invention is a hybrid of domains in which each domain has a different origin derived from immunoglobulin selected from the group consisting of IgG, IgA, IgD, IgE, and IgM.

In still another specific embodiment, the immunoglobulin Fc fragment applied to the method according to the present invention is a dimer or a multimer consisting of single chain immunoglobulins of the same origin.

In still another specific embodiment, the immunoglobulin Fc fragment applied to the method according to the present invention is a human aglycosylated IgG4 Fc fragment.

In still another specific embodiment, the step of conjugating according to the present invention includes linking a physiologically active protein or peptide to an immunoglobulin Fc fragment via a non-peptidyl polymer.

In still another specific embodiment, the physiologically active protein or peptide conjugated with the immunoglobulin Fc fragment prepared by the method according to the present invention is in the form of a fusion protein.

In still another specific embodiment, the non-peptidyl polymer applied to the method according to the present invention is polyethylene glycol.

In still another specific embodiment, the non-peptidyl polymer applied to the method according to the present invention is selected from the group consisting of polypropylene glycol, copolymer of ethylene glycol and propylene glycol, polyoxyethylated polyol, polyvinyl alcohol, polysaccharide, dextran, polyvinyl ethyl ether, a biodegradable polymer such as polylactic acid (PLA) and polylactic-glycolic acid (PLGA), a lipid polymer, chitin, hyaluronic acid, and a combination thereof.

In still another specific embodiment, the method according to the present invention is to improve the solubility of the physiologically active protein or peptide in an aqueous solution.

In still another specific embodiment, the aqueous solution applied to the method according to the present invention includes a citric acid or acetic acid buffer solution, polysorbate as a non-ionic surfactant, mannitol as a sugar alcohol, and sodium chloride or methionine as an isotonic agent.

In still another specific embodiment, the aqueous solution applied to the method according to the present invention has a pH from 5.0 to 7.0.

In still another specific embodiment, the physiologically active protein or peptide in the method according to the present invention is exendin-4, glucagon, glucagon-like peptide-1 (GLP-1), glucagon-like peptide-2 (GLP-2), parathyroid hormone (PTH), calcitonin, insulin, or oxyntomodulin.

In still another specific embodiment, the aqueous solution applied to the method according to the present invention further includes a surfactant.

Another aspect provides a composition for improving the solubility of a physiologically active protein or peptide, including an immunoglobulin Fc fragment, in which the composition improves the solubility compared to a composition without an immunoglobulin Fc fragment.

In a specific embodiment, the composition includes a conjugate of physiologically active protein or peptide and an immunoglobulin Fc fragment in which a physiologically active protein or peptide is linked to an immunoglobulin Fc fragment via a peptidyl linker or a non-peptidyl linker as an active ingredient.

### [Mode for Invention]

The present invention provides a method for improving the solubility of a physiologically active protein or peptide compared to that of a physiologically active protein or peptide which is not conjugated to an immunoglobulin Fc fragment, in which the method includes conjugating the physiologically active protein or peptide to an immunoglobulin Fc fragment. The method of the present invention shows an effect of improving the solubility of the physiologically active protein or peptide even without addition of a surfactant to a solvent. In the case of the physiologically active protein or peptide, specifically, insulin, oxyntomodulin, etc., which is a component of the known protein drugs, it shows low solubility in the pH range from 5 to 7, which is the general pH range of formulations for body administration. Accordingly, there is a difficulty in that the physiologically active protein or peptide is prepared into formulations at a desired concentration. There is also a problem in that injectable formulations thereof may cause precipitation in the body to generate unintended side effects. Surprisingly, the present invention confirmed that when the physiologically active protein or peptide is conjugated with an immunoglobulin Fc fragment, it shows increased solubility in the above pH range. That is, the method of the present invention has an effect of improving solubility of the peptide or protein, such as insulin or oxyntomodulin, which shows low solubility in the weak acidic pH range of 5 to 7.

As used herein, the term "solubility" refers to the extent to which the physiologically active protein or peptide is dissolved in a solvent appropriate for human body administration. In detail, it refers to a saturation extent of the solute in a given solvent at a specific temperature. Solubility may be measured by determining a solute concentration at the point of saturation. For example, the concentration may be measured using a UV spectrophotometer or HPLC after adding an excessive amount of the solute to a solvent, and then stirring and filtering the solution, but is not limited thereto. To use the physiologically active protein or peptide for treatment, it is important to dissolve a pharmaceutically effective amount thereof in a solvent, which is an important factor that should be considered in determining concentration of the lyophilized formulation upon reconstitution or concentration of an active ingredient of a liquid formulation.

It was confirmed that when the physiologically active protein or peptide is conjugated with an immunoglobulin Fc fragment, its solubility is remarkably increased compared to a physiologically active protein or peptide to which the fragment is not linked. With this as a basis, the present invention is characterized in that solubility of the physiologically active protein or peptide is improved in an aqueous solution.

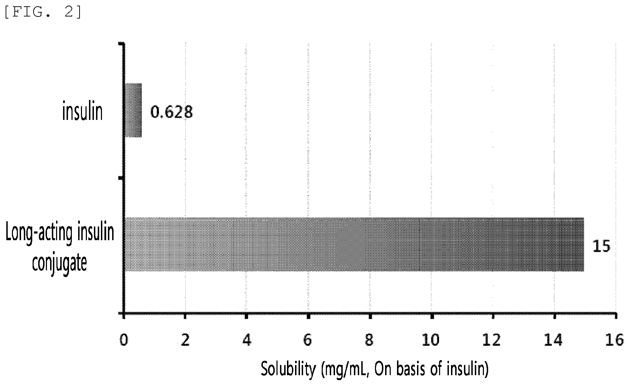

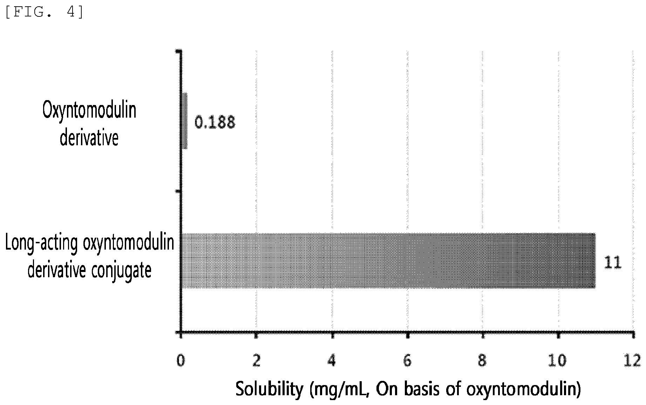

According to an embodiment of the present invention, when insulin or oxyntomodulin was used as a representative physiologically active peptide and immunoglobulin Fc was conjugated thereto, solubility of insulin and oxyntomodulin was remarkably increased (FIGS. 2 and 4).

As used herein, the term "aqueous solution" refers to a solution that is intended to dissolve the physiologically active protein or peptide. The aqueous solution is a solution suitable for administration of the physiologically active protein or peptide to the human body. In a specific embodiment of the present invention, the aqueous solution may have a neutral or weak acidic pH, for example, a pH of 5.0 to 7.0. The aqueous solution applied to the method of the present invention is not limited to a particular type, but it may have a citric acid or acetic acid buffer solution containing polysorbate as a non-ionic surfactant, mannitol as a sugar alcohol, and sodium chloride or methionine as an isotonic agent.

In the method according to the present invention, the immunoglobulin Fc fragment may be linked to the physiologically active protein or peptide via a non-peptidyl polymer. Specifically, the immunoglobulin Fc fragment may be linked to the non-peptidyl polymer which is linked to the physiologically active protein or peptide, or an immunoglobulin Fc fragment-non-peptidyl polymer may be linked to the physiologically active protein or peptide.

As used herein, the term "non-peptidyl polymer" refers to a biocompatible polymer including one or more repeating units linked to each other by a covalent bond excluding a peptide bond.

The non-peptidyl polymer which can be used in the present invention may be selected from the group consisting of polyethylene glycol, polypropylene glycol, copolymer of ethylene glycol and propylene glycol, polyoxyethylated polyols, polyvinyl alcohol, polysaccharides, dextran, polyvinyl ethyl ether, biodegradable polymers such as PLA (polylactic acid) and PLGA (polylactic-glycolic acid), lipid polymers, chitins, hyaluronic acid, and combinations thereof, and preferably, polyethylene glycol, but is not limited thereto. Also, derivatives thereof which are well known in the art and easily prepared within the skill of the art are included in the scope of the present invention.

Any non-peptidyl polymer may be used without limitation, as long as it is a polymer that has a resistance to *in vivo* proteolytic enzymes, and thus is not easily cleaved by proteolytic enzymes to allow the immunoglobulin Fc fragment to function as a carrier. The non-peptidyl polymer has a molecular weight in the range of 1 kDa to 100 kDa, and specifically, of 1 kDa to 20 kDa. Also, the non-peptidyl polymer of the present invention, linked to the immunoglobulin Fc fragment, may be one polymer or a combination of different types of polymers.

The non-peptidyl polymer may have a reactive group capable of binding to the immunoglobulin Fc fragment and the protein or peptide drug, and thus functions as a linker to link the immunoglobulin Fc fragment and the protein or peptide.

The linking of the physiologically active protein or peptide with the immunoglobulin Fc fragment using the non-peptidyl polymer as a linker may be achieved by performing (1) reacting the non-peptidyl polymer with any one of the physiologically active protein or peptide and the immunoglobulin Fc fragment; and (2) reacting the reaction product of step (1) with the other one of the physiologically active protein or peptide and the immunoglobulin Fc fragment, sequentially.

However, if the immunoglobulin Fc fragment is linked to the non-peptidyl polymer having a reactive group at both ends in step (1), a part of the products of step (1) may be in the form of a bridge in which two N-terminals of immunoglobulin Fc fragment are linked to both ends of the non-peptidyl polymer under specific reaction conditions. The products of step (1) in the form of a bridge may prevent a secondary coupling reaction of linking the physiologically active protein or peptide thereto or may greatly reduce a coupling yield. In an embodiment, therefore, in order to prevent the bridge formation between the immunoglobulin Fc fragment and the immunoglobulin Fc fragment, the non-peptidyl polymer and the physiologically active protein or peptide may be first reacted, and then the non-peptidyl polymer-linked physiologically active protein or peptide may be reacted with the immunoglobulin Fc fragment, sequentially. However, there is no need to perform the method of improving solubility of the present invention in this order.

In step (1), a chemical reaction for linking the non-peptidyl polymer to the physiologically active protein or peptide or the immunoglobulin Fc fragment may be performed by a known method. For example, the non-peptidyl polymer having a reactive group at both ends may be reacted with the physiologically active protein or peptide or the immunoglobulin Fc fragment at 0°C to 25°C for 1 hour to 16 hours. Through this reaction, the physiologically active protein or peptide or the immunoglobulin Fc fragment may be covalently linked to one non-peptidyl polymer via the reactive group.

The reaction of linking the physiologically active protein or peptide to the non-peptidyl polymer in step (1) may be performed in a reaction solution containing an organic solvent. In this regard, the organic solvent may be any organic solvent generally used in the art, but is not limited to, specifically a primary, secondary, or tertiary alcohol, and an alcohol having 1 to 10 carbon atoms. More specifically, the alcohol may be isopropanol, ethanol, or methanol. The organic solvent may be freely selected from organic solvents suitable for the type of the physiologically active protein or peptide. Further, the organic solvent may be used for linkage between the physiologically active protein or peptide and the non-peptidyl polymer, but is not limited to, is specifically included in an amount of 10% to 60%, more specifically, in an amount of 30% to 55 %, and much more specifically, in an amount of 45% to 55%, based on a total amount of the reaction solution. Further, the pH of the reaction solution may be, but is not limited to, specifically 4.5 to 7.0 and more specifically 5.0 to 6.5.

Further, according to the type of the reactive group participating in the reaction, a reducing agent may be further included to perform the above steps.

Specifically, the reducing agent of the present invention refers to any reducing agent known in the art which functions to reduce a reversible imine double bond produced from bonding between the aldehyde group of the non-peptidyl polymer and an amine group of the polypeptide (physiologically active protein or peptide, immunoglobulin Fc fragment) to form a covalent bond. With respect to the objects of the present invention, the reducing agent may be included in the reaction solution in order to allow the covalent bonding between the non-peptidyl polymer and the physiologically active protein or peptide or the immunoglobulin Fc fragment. The reducing agent of the present invention may be any reducing agent known in the art, but is not limited to, preferably sodium cyanoborohydride, borane pyridine complex, sodium borohydride, borane dimethylamine complex, borane trimethylamine complex, or sodium triacetoxyborohydride. An adequate reducing agent may be selected depending on the kinds of the physiologically active polypeptide or the immunoglobulin constant region and the reaction solvent.

Further, the reducing agent may be included at a final concentration of 1 mM to 20 mM for a linkage reaction (reaction of step (1)) between the physiologically active protein or peptide and the non-peptidyl polymer, or the immunoglobulin Fc fragment and the non-peptidyl polymer, and at a final concentration of 1 mM to 100 mM for the coupling reaction (reaction of step (2)).

Meanwhile, the non-peptidyl polymer may have a reactive group at both ends, each reactive group capable of binding to the immunoglobulin Fc fragment and the physiologically active protein or peptide, specifically, each reactive group capable of binding to an amine group of the N-terminus or lysine, or a thiol group of cysteine of the physiologically active protein or peptide; or the immunoglobulin Fc fragment. The reactive group at both ends is specifically selected from the group consisting of a reactive aldehyde group, a propionaldehyde group, a butyraldehyde group, a maleimide group, and a succinimide derivative. In this regard, the succinimide derivative may be succinimidyl carboxymethyl, succinimidyl valerate, succinimidyl methylbutanoate, succinimidyl methylpropionate, succinimidyl butanoate, succinimidyl propionate, N-hydroxysuccinimide, or succinimidyl carbonate, but is not limited thereto. Any reactive group may be used without limitation, as long as it binds to the amine group or thiol group of an amino acid residue of the immunoglobulin Fc fragment and the physiologically active protein or peptide.

In particular, when the non-peptidyl polymer has a reactive aldehyde group at both ends, it is effective in linking with the physiologically active protein or peptide and the immunoglobulin at both ends with minimal nonspecific reactions. A final product generated by reductive alkylation by an aldehyde bond is much more stable than when linked by an amide bond. The aldehyde reactive group selectively binds to the N-terminus at a low pH, and may bind to a lysine residue (Lys) to form a covalent bond at a high pH, for example, at a pH of 9.0.

The reactive groups at both ends of the linker as the non-peptidyl polymer may be the same as or different from each other. For example, the linker may possess a maleimide group at one end, and an aldehyde group, a propionaldehyde group, or a butyl aldehyde group at the other end. When a polyethylene glycol having a reactive hydroxyl group at both ends thereof is used as the non-peptidyl polymer, the hydroxyl group may be activated into various reactive groups by known chemical reactions, or a commercially available polyethylene glycol having a modified reactive group may be used to prepare a long-acting protein conjugate of the present invention.

Further, the reactive groups of the non-peptidyl polymer may bind to the N-terminal amine group or the side-chain amine group of the lysine residue of the physiologically active protein or peptide and the immunoglobulin Fc fragment, respectively, but are not particularly limited thereto. In this regard, the lysine residue on the physiologically active protein or peptide and the immunoglobulin Fc fragment is not limited to a particular position, and non-limiting examples of the lysine residue also include non-natural amino acids and Lys derivatives, as long as they have the amine group capable of binding to the reactive group of the non-peptidyl polymer, as well as a natural lysine.

Meanwhile, when the physiologically active protein or peptide is first reacted with the non-peptidyl polymer in step (1), the physiologically active protein or peptide and the non-peptidyl polymer are reacted at a molar ratio of 1:1 to 1:20, and in step (2), the conjugate of the physiologically active protein or peptide and the non-peptidyl polymer which is a product of step (1) is reacted with the immunoglobulin Fc fragment at a molar ratio of 1:0.5 to 1:10, but is not limited thereto.

In addition, the method of the present invention may include a process of separating and purifying the physiologically active polypeptide-non-peptidyl polymer conjugate or the immunoglobulin Fc fragment-non-peptidyl polymer conjugate which is the product of step (1) prior to step (2) after step (1). This process may be performed using various separation methods, such as chromatography, known in the art.

In step (2), when the physiologically active polypeptide-non-peptidyl polymer conjugate is reacted with the immunoglobulin Fc fragment, a pH condition is, but not particularly limited to, a pH of 4.0 to 9.0.

Meanwhile, the physiologically active protein or peptide and the immunoglobulin Fc fragment may be conjugated to each other in the form of a fusion protein.

As used herein, the term "fusion protein" refers to a protein created through the joining of two or more genes which originally coded for separate proteins. The fusion protein may be prepared artificially by recombinant DNA technology. Further, the fusion protein may be in the form in which the physiologically active protein or peptide and the immunoglobulin Fc fragment are linked to each other via no linker or a peptidyl linker.

Herein, the peptidyl linker refers to a peptide linking two kinds of proteins constituting the fusion protein. The peptidyl linker may be included in order to independently fold the two proteins constituting the fusion protein or to maintain each function of two proteins even in the form of the fusion protein, but is not limited thereto.

As used herein, the term "immunoglobulin Fc fragment" refers to the heavy-chain constant region 2 (CH2) and the heavy-chain constant region 3 (CH3) of an immunoglobulin, excluding the variable regions of the heavy and light chains, the heavy-chain constant region 1 (CH1) and the light-chain constant region 1 (CL1) of an immunoglobulin, and it may include a hinge region at the heavy-chain constant region. Further, the immunoglobulin Fc fragment of the present invention may be an extended immunoglobulin Fc fragment including a part or all of heavy-chain constant region 1 (CH1) and/or the light-chain constant region 1 (CL1), except for the variable regions of the heavy and light chains of an immunoglobulin, as long as it has an effect substantially similar to or better than that of the native protein. Also, it may be a region having a deletion in a relatively long portion of the amino acid sequence of CH2 and/or CH3. That is, the immunoglobulin Fc fragment of the present invention may include (1) a CH1 domain, a CH2 domain, a CH3 domain and a CH4 domain, (2) a CH1 domain and a CH2 domain, (3) a CH1 domain and a CH3 domain, (4) a CH2 domain and a CH3 domain, (5) a combination of one or more domains of the constant region and an immunoglobulin hinge region (or a portion of the hinge region), and (6) a dimer of each domain of the heavy-chain constant regions and the light-chain constant region.

With respect to the objects of the present invention, the immunoglobulin Fc fragment refers to a substance that has an effect of increasing solubility of the drug binding thereto, that is, the physiologically active protein or peptide, and it may also refer to a drug carrier.

As used herein, the term "carrier" refers to a substance that binds together with a drug. Generally, the carrier binds to a drug to increase or eliminate physiological activity of the drug. However, with respect to the object of the present invention, the carrier of the present invention refers to a substance that minimizes a reduction in physiological activity of the drug and increases *in vivo* stability of the drug and duration of efficacy by retarding delivery of the drug from the site of administration to the blood upon subcutaneous administration, and also improves solubility of the protein or peptide at the same time. As the drug carrier, various substances such as lipids, polymers, etc. have been studied, but the present invention provides the immunoglobulin Fc fragment as the carrier for improving *in vivo* duration of the drug to which the carrier is linked, minimizing a reduction in the activity *in vivo,* and also maximizing the solubility. Further, the immunoglobulin Fc fragment is a biodegradable polypeptide which can be metabolized *in vivo,* so that it can safely be used as a drug carrier. Owing to the characteristics, the conjugate in which the immunoglobulin Fc fragment is linked to the physiologically active protein or peptide may be designated as a long-acting conjugate in the present invention.

Further, the immunoglobulin Fc fragment used to improve solubility of the physiologically active protein or peptide in the present invention may be linked to the physiologically active protein or peptide via a linker or directly. In this regard, the site of physiologically active protein or peptide to which the immunoglobulin Fc fragment is linked is not particularly limited, and any site within the protein or peptide or at the N-terminus or C-terminus is possible. However, to allow the conjugate of the physiologically active protein or peptide and the immunoglobulin Fc fragment which is prepared by the method of the present invention to exhibit physiological activity when administered to the human body, the site not inhibiting the physiological activity is preferably selected according to the type of the physiologically active protein or peptide to be applied, but is not limited thereto.

In addition, the immunoglobulin Fc fragment is more advantageous in terms of production, purification, and yield of the conjugate than an entire immunoglobulin molecule, owing to its relatively low molecular weight. Further, since it is devoid of Fab, which exhibits high non-homogeneity due to the difference in amino acid sequence from one antibody to another, it is expected to significantly enhance homogeneity and to reduce the possibility of inducing blood antigenicity.

Further, the immunoglobulin Fc fragment of the present invention includes not only the native amino acid sequence but also sequence derivatives thereof. The amino acid sequence derivative means that it has one or more amino acid residues different from the wild-type amino acid sequence, and it may occur naturally or may be artificially generated. The immunoglobulin Fc fragment includes derivatives as a result of deletion, insertion, conserved or non-conserved substitution, or a combination thereof. An insertion is generally made by the addition of a consecutive amino acid sequence of about 1 to 20 amino acids, or may be made with a longer sequence. A deletion is generally in the range of about 1 to 30 amino acid residues. The techniques of preparing the sequence derivatives of the immunoglobulin Fc fragment are disclosed in International Patent Publication Nos. WO 97/34631 and WO 96/32478, which are incorporated herein by reference. Amino acid exchanges in proteins and peptides, which do not generally alter the activity of the molecules, are known in the art (H. Neurath, R.L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn , Leu/Ile, Leu/Val, Ala/Glu, and Asp/Gly, in both directions. In addition, the immunoglobulin Fc fragment, if desired, may be modified by phosphorylation, sulfation, acrylation, glycosylation, methylation, farnesylation, acetylation, amidation, and the like.

The above-described immunoglobulin Fc derivative may be a derivative which has a biological activity equivalent to that of the immunoglobulin Fc fragment of the present invention, but has increased structural stability of the immunoglobulin Fc fragment against heat, pH, etc.

Further, the immunoglobulin Fc fragment may be obtained from a native type isolated from humans or animals such as cows, goats, pigs, mice, rabbits, hamsters, rats, guinea pigs, etc., or may be recombinants or derivatives thereof obtained from transformed animal cells or microorganisms. Herein, they may be obtained from a native immunoglobulin by isolating whole immunoglobulins from human or animal organisms and treatment thereof with a proteolytic enzyme. Papain digests the native immunoglobulin into Fab and Fc regions, and pepsin treatment results in the production of pF'c and F(ab)₂ fragments. These fragments may be subjected, for example, to size exclusion chromatography to isolate Fc or pF'c. Specifically, the immunoglobulin Fc fragment according to the present invention may be a recombinant human-derived immunoglobulin Fc fragment that is obtained from a microorganism.

In addition, the immunoglobulin Fc region of the present invention may be in the form of having native sugar chains, increased sugar chains compared to a native form or decreased sugar chains compared to the native form, or may be in a deglycosylated form. Increased or decreased glycosylation or deglycosylation of the immunoglobulin Fc may be achieved by typical methods, for example, by using a chemical method, an enzymatic method, or a genetic engineering method using microorganisms. Herein, when deglycosylated, the complement (C1q) binding to an immunoglobulin Fc fragment becomes significantly decreased and antibody-dependent cytotoxicity or complement-dependent cytotoxicity is reduced or removed, thereby not inducing unnecessary immune responses *in vivo.* In this context, deglycosylated or aglycosylated immunoglobulin Fc fragments are more consistent with the purpose of drug carriers.

As used herein, the term "deglycosylation" refers to the enzymatic removal of sugar moieties from an immunoglobulin Fc fragment, and the term "aglycosylation" means that an immunoglobulin Fc fragment is produced in an unglycosylated form by a prokaryote, specifically *E. coli.*

Meanwhile, the immunoglobulin Fc fragment may be derived from humans or other animals including cows, goats, swine, mice, rabbits, hamsters, rats, and guinea pigs, and preferably humans. In addition, the immunoglobulin Fc fragment may be an Fc fragment that is derived from IgG, IgA, IgD, IgE, and IgM, or that is made by combinations thereof or hybrids thereof. Specifically, it is derived from IgG or IgM, which are among the most abundant proteins in human blood, and most specifically from IgG, which is known to enhance the half-lives of ligand-binding proteins.

Meanwhile, as used herein, the term "combination" means that polypeptides encoding single-chain immunoglobulin Fc fragments of the same origin are linked to a single-chain polypeptide of a different origin to form a dimer or multimer. That is, a dimer or multimer may be formed from two or more fragments selected from the group consisting of IgG Fc, IgA Fc, IgM Fc, IgD Fc, and IgE Fc fragments.

The term "hybrid", as used herein, means that sequences encoding two or more immunoglobulin Fc fragment of different origin are present in a single-chain immunoglobulin Fc fragment. In the present invention, various types of hybrids are possible. That is, domain hybrids may be composed of one to four domains selected from the group consisting of CH1, CH2, CH3, and CH4 of IgG Fc, IgM Fc, IgA Fc, IgE Fc, and IgD Fc, and may include the hinge region.

Meanwhile, IgG is divided into IgG1, IgG2, IgG3, and IgG4 subclasses, and the present invention includes combinations and hybrids thereof. Preferred are IgG2 and IgG4 subclasses, and most preferred is the Fc fragment of IgG4 rarely having effector functions such as complement dependent cytotoxicity (CDC).

The immunoglobulin Fc fragment of the present invention may be in the recombinant form in which a hinge region is linked to the N-terminus thereof. In this case, the immunoglobulin Fc fragments expressed as aggregates do not cause loss of activity. Also, these are Fc fragments in the form of an active dimer or monomer having no initiation methionine residue encoded by an initiation codon, and therefore, there are advantages of solubilization and refolding.

The appropriate immunoglobulin Fc fragment in the present invention is described in detail in Korean Patent Nos. 755315, 775343, and 824505, which are incorporated herein by reference.

That is, the most preferable immunoglobulin Fc fragment used as the drug carrier for fusion protein with insulin and oxyntomodulin according to the present invention is a human IgG4-derived non-glycosylated Fc region. The human-derived Fc fragment is more preferable than a non-human-derived Fc fragment, which may act as an antigen in the human body and cause undesirable immune responses such as the production of a new antibody against the antigen.

As used herein, the term "physiologically active protein or peptide" refers to a protein or peptide regulating gene expression or physiological functions. With respect to the objects of the present invention, any physiologically active protein or peptide may be included without limitation, as long as it increases solubility.

Meanwhile, amino acids mentioned herein are abbreviated according to the nomenclature rules of IUPAC-IUB as follows:

| | | | |
|---|---|---|---|
| Alanine: | Ala or A | Arginine: | Arg or R |
| Asparagine: | Asn or N | Aspartic acid: | Asp or D |
| Cysteine: | Cys or C | Glutamic acid: | Glu or E |
| Glutamine: | Gln or Q | Glycine: | Gly or G |
| Histidine: | His or H | Isoleucine: | Ile or I |
| Leucine: | Leu or L | Lysine: | Lys or K |
| Methionine: | Met or M | Phenylalanine: | Phe or F |
| Proline: | Pro or P | Serine: | Ser or S |
| Threonine: | Thr or T | Tryptophan: | Trp or W |
| Tyrosine: | Tyr or Y | Valine: | Val or V |

Further, throughout the present specification, one- and three-letter codes may be used for the amino acids.

Further, the physiologically active protein or peptide is a concept encompassing all of derivatives, variants, and fragments thereof, in addition to the native physiologically active protein or peptide.

As used herein, the term "derivative" refers to a peptide having chemical substitution (e.g., alpha-methylation, alpha-hydroxylation), deletion (e.g., deamination), or modification (e.g., N-methylation) of some groups of amino acid residues in the amino acid sequence of the native physiologically active protein or peptide. The derivative may include a peptide mimetic retaining the activity of the native physiologically active peptide without sequence homology with the native physiologically active peptide.

As used herein, the term "peptide mimetic" refers to a protein-like chain which is designed to mimic the peptide. The peptide mimetic may be exemplified by a D-peptide mimetic containing a D-amino acid, but is not limited thereto. The peptide mimetic may be easily prepared by using a technique of preparing peptide mimetics known in the art.

As used herein, the term "variant" refers to a protein or peptide having one or more amino acid sequences different from those of the native protein or peptide, and refers to a peptide that retains the activity of the native protein. The variant may be prepared by any one of substitution, addition, deletion, and modification or by a combination thereof in a part of the amino acid sequences of the native protein or peptide. Herein, the added amino acids may be non-naturally occurring amino acids (e.g., D-type amino acids).

As used herein, the term "fragment" refers to a fragment having one or more amino acids deleted at the N-terminus or the C-terminus of the native protein or peptide, and preferably, a fragment having the activity of the native protein.

The physiologically active protein or peptide may be prepared by any method derived from a natural or recombinant origin, and for example, it may be a recombinant protein prepared by using a prokaryotic cell such as *E. coli* as a host cell, but is not limited thereto.

Meanwhile, the physiologically active protein or peptide may be oxyntomodulin, exendin-4, glucagon-like peptide-1 (GLP-1), GLP-2, insulinotropic peptides such as glucose-dependent insulinotropic peptide (GIP), insulin, glucagon, parathyroid hormone (PTH), or calcitonin, but is not limited thereto. As described above, the physiologically active protein or peptide is a concept encompassing all of derivatives, variants, and fragments thereof, in addition to the native physiologically active protein or peptide.

As used herein, the term "insulin" refers to a peptide that is secreted by the pancreas in response to elevated glucose levels in the blood to take up glucose in the liver, muscle, or adipose tissue and turn it into glycogen, and to stop the use of fat as an energy source, and thus controls the blood glucose level. This peptide includes the native insulin, basal insulin, insulin agonists, precursors, derivatives, fragments thereof, and variants thereof.

As used herein, the term "native insulin" refers to a hormone that is secreted by the pancreas to promote glucose absorption and inhibit fat breakdown, and thus functions to control the blood glucose level. Insulin is formed from a precursor having no function of regulating the blood glucose level, known as proinsulin, through processing. The amino acid sequences of insulin are as follows:
A chain: 
Gly-Ile-Val-Glu-Gln-Cys-Cys-Thr-Ser-Ile-Cys-Ser-Leu-
Tyr-Gln-Leu-Glu-Asn-Tyr-Cys-Asn (SEQ ID NO: 1)
B chain:

Phe-Val-Asn-Gln-His-Leu-Cys-Gly-Ser-His-Leu-Val-Glu-
Ala-Leu-Tyr-Leu-Val-Cys-Gly-Glu-Arg-Gly-Phe-Phe-Tyr-Thr-Pro-
Lys-Thr (SEQ ID NO: 2)

As used herein, the term "basal insulin" refers to a peptide that manages normal daily blood glucose level, for example, levemir, lantus, deglude, etc.

As used herein, the term "insulin agonist" refers to a compound that binds to the insulin receptor to show the biological activity equal to that of insulin, which is irrelevant to the structure of insulin.

The term "insulin variant" refers to a peptide having one or more amino acid sequences different from those of native insulin, and retaining the function of controlling the blood glucose level in the body.

The term "insulin derivative" refers to a peptide having an amino acid sequence homology with the native insulin of at least 80%, which may have some groups on the amino acid residue chemically substituted, deleted, or modified, and has a function of regulating the blood glucose level in the body. The "insulin fragment" refers to a fragment having one or more amino acids deleted at the N-terminus or the C-terminus of insulin and having a function of regulating the blood glucose level in the body.

Each of the preparation methods for the agonists, derivatives, fragments, and variants of insulin may be used individually or in combination. For example, the present invention includes a peptide that has one or more amino acids different from those of the native peptide and deamination of the N-terminal amino acid residue, and has a function of regulating the blood glucose level in the body. The insulin used in the present invention may be produced by a recombination technology, and may also be synthesized using a solid phase synthesis method.

Further, the insulin used in the present invention may be linked to a non-peptidyl polymer. This non-peptidyl polymer may be used as a linker in the present invention. The non-peptidyl polymer is used as a linker to link the immunoglobulin Fc fragment, thereby maintaining the activity of insulin and improving the solubility, and also improving the stability. Application of a peptide linker using a genetic recombination technology is possible.

Further, the present invention is characterized in that the physiologically active protein or peptide is conjugated with an immunoglobulin Fc fragment to improve solubility of the protein or peptide. There is no particular limitation in the binding site. However, modification of the A chain of insulin may reduce the activity and stability, and therefore, the immunoglobulin Fc fragment may be linked to the B chain via a linker or no linker. In particular, the immunoglobulin Fc fragment may be linked to the N-terminus of the insulin B chain via a non-peptidyl linker or no non-peptidyl linker, but is not particularly limited thereto.

As used herein, the term "oxyntomodulin" refers to a peptide derived from a glucagon precursor, pre-glucagon, and includes native oxyntomodulin, precursors, derivatives, fragments, and variants thereof.

Specifically, the oxyntomodulin has an amino acid sequence of HSQGTFTSDYSKYLDSRRAQDFVQWLMNTKRNRNNIA (SEQ ID NO: 3) .

The oxyntomodulin derivative includes peptides, peptide derivatives, or peptide mimetics that are prepared by addition, deletion, or substitution of a part of the amino acids of the oxyntomodulin sequence so as to activate both of the GLP-1 receptor and the glucagon receptor at a high level compared to the native oxyntomodulin. In the present invention, the oxyntomodulin derivative may have any one amino acid sequence of SEQ ID NOS: 4 to 36.

Further, the oxyntomodulin fragment retains the function of controlling the blood glucose level in the body.

Further, the oxyntomodulin variant is a peptide that has one or more amino acid residues different from those of the amino acid sequence of native oxyntomodulin and possesses a function of activating GLP-1 and glucagon receptors. Methods of preparing the oxyntomodulin variant, derivative, and fragment may be used alone or in combination. For example, the present invention includes a peptide that has one or more amino acids different from those of native peptide and deamination of the N-terminal amino acid residues, and has a function of activating both GLP-1 receptor and glucagon receptor.

In the present invention, the oxyntomodulin derivative encompasses any peptide that is prepared by substitutions, additions, deletions, or post-translational modifications (e.g., methylation, acylation, ubiquitination, intramolecular covalent bonding) in the amino acid sequence of SEQ ID NO: 3 so as to activate the glucagon and GLP-1 receptors at the same time. Upon substitution or addition of amino acids, any of the 20 amino acids commonly found in human proteins, as well as atypical or non-naturally occurring amino acids, may be used. Commercial sources of atypical amino acids include Sigma-Aldrich, ChemPep Inc., and Genzyme Pharmaceuticals. The peptides including these amino acids and atypical peptide sequences may be synthesized and purchased from commercial suppliers, for example, American Peptide Company (USA), Bachem (USA), or Anygen (Korea).

In a specific embodiment, the oxyntomodulin derivative of the present invention is a novel peptide including the amino acid sequence of the following Formula 1:

R1-X1-X2-GTFTSD-X3-X4-X5-X6-X7-X8-X9-X10-X11-X12-X13- X14-X15-X16-X17-X18-X19-X20-X21-X22-X23-X24-R2 (Formula 1)

wherein R1 is histidine, desamino-histidyl, dimethyl-histidyl (N-dimethyl-histidyl), beta-hydroxyimidazopropionyl, 4-imidazoacetyl, beta-carboxyimidazopropionyl, or tyrosine;
X1 is AIB (aminoisobutyric acid), D-alanine, glycine, Sar (N-methyl glycine), serine, or D-serine;
X2 is glutamic acid or glutamine;
X3 is leucine or tyrosine;
X4 is serine or alanine;
X5 is lysine or arginine;
X6 is glutamine or tyrosine;
X7 is leucine or methionine;
X8 is aspartic acid or glutamic acid;
X9 is glutamic acid, serine, alpha-methyl-glutamic acid, or deleted;
X10 is glutamine, glutamic acid, lysine, arginine, serine, or deleted;
X11 is alanine, arginine, valine, or deleted;
X12 is alanine, arginine, serine, valine, or deleted;
X13 is lysine, glutamine, arginine, alpha-methyl-glutamic acid, or deleted;
X14 is aspartic acid, glutamic acid, leucine, or deleted;
X15 is phenylalanine or deleted;
X16 is isoleucine, valine, or deleted;
X17 is alanine, cysteine, glutamic acid, lysine, glutamine, alpha-methyl-glutamic acid, or deleted;
X18 is tryptophan or deleted;
X19 is alanine, isoleucine, leucine, serine, valine, or deleted;
X20 is alanine, lysine, methionine, glutamine, arginine, or deleted;
X21 is asparagine or deleted;
X22 is alanine, glycine, threonine, or deleted;
X23 is cysteine, lysine, or deleted;
X24 is a peptide having 2 to 10 amino acids consisting of combinations of alanine, glycine, and serine, or deleted; and
R2 is KRNRNNIA (SEQ ID NO: 37), GPSSGAPPPS (SEQ ID NO: 38), GPSSGAPPPSK (SEQ ID NO: 39), HSQGTFTSDYSKYLD (SEQ ID NO: 40), HSQGTFTSDYSRYLDK (SEQ ID NO: 41), HGEGTFTSDLSKQMEEEAVK (SEQ ID NO: 42), or deleted (excluded if the amino acid sequence of Formula 1 is identical to that of SEQ ID NO: 3).

In order to enhance the activity of the wild-type oxyntomodulin for the glucagon receptor and the GLP-1 receptor, the oxyntomodulin derivative of the present invention may be substituted with 4-imidazoacetyl where the alpha-carbon of histidine at position 1 of the amino acid sequence represented by SEQ ID NO: 3 is deleted, desamino-histidyl where the N-terminal amino group is deleted, dimethyl-histidyl (N-dimethyl-histidyl) where the N-terminal amino group is modified with two methyl groups, beta-hydroxyimidazopropionyl where the N-terminal amino group is substituted with a hydroxyl group, or beta-carboxyimidazopropionyl where the N-terminal amino group is substituted with a carboxyl group. In addition, the GLP-1 receptor-binding region may be substituted with amino acids that enhance hydrophobic and ionic bonds or combinations thereof. A part of the oxyntomodulin sequence may be substituted with the amino acid sequence of GLP-1 or exendin-4 to enhance the activity on GLP-1 receptor.

Further, a part of the oxyntomodulin sequence may be substituted with a sequence stabilizing an alpha helix. For example, amino acids at positions 10, 14, 16, 20, 24, and 28 of the amino acid sequence of Formula 1 may be substituted with amino acids consisting of Tyr(4-Me), Phe, Phe(4-Me), Phe(4-Cl), Phe(4-CN), Phe(4-NO₂), Phe(4-NH₂), Phg, Pal, Nal, Ala(2-thienyl), and Ala(benzothienyl) that are known to stabilize an alpha helix, or amino acid derivatives.

Herein, Tyr(4-Me) is a tyrosine derivative in which hydrogen of a hydroxyl group of tyrosine is substituted with a methyl group; Phe(4-Me) is a phenylalanine derivative in which the para-position of phenylalanine is substituted with a methyl group; Phe(4-Cl) is a phenylalanine derivative in which the para-position of phenyl group is substituted with chloride; Phe(4-CN) is a phenylalanine derivative in which the para-position of the phenyl group is substituted with a cyanide group; Phe(4-NO₂) is a phenylalanine derivative in which the para-position of the phenyl group is substituted with a nitro group; and Phe(4-NH₂) is a phenylalanine derivative in which the para-position of the phenyl group is substituted with an amine group.

Further, Phg represents phenylglycine; Pal is an alanine derivative in which the beta-methyl group of alanine is substituted with a pyridyl group; Nal is an alanine derivative in which the beta-methyl group of alanine is substituted with a naphthyl group; Ala(2-thienyl) is an alanine derivative in which the beta-methyl group of alanine is substituted with a 2-thienyl group; and Ala(benzothienyl) is an alanine derivative in which the beta-methyl group of alanine is substituted with a benzo-thienyl group.

There are no limitations on the type and number of alpha helix-stabilizing amino acids or amino acid derivatives to be inserted. In a specific embodiment, intramolecular rings may be formed between amino acid residues. For example, intramolecular rings may be formed at positions 10 and 14, 12 and 16, 16 and 20, 20 and 24, and 24 and 28, and in this case, the corresponding positions may be replaced by a pair of glutamic acid and lysine. There is no particular limitation in the number of intramolecular rings formed in the oxyntomodulin derivative, of which solubility may be improved by the method of the present invention.

In an embodiment, oxyntomodulin, of which solubility is improved by the method of the present invention, or a derivative thereof may be an oxyntomodulin derivative having an amino acid sequence selected from the following Formulae 2 to 6. In a specific embodiment, the oxyntomodulin derivative of the present invention is a peptide having an amino acid sequence of the following Formula 2, in which the amino acid sequence of oxyntomodulin is substituted with the sequence of exendin or GLP-1:

R1-A'-R3 (Formula 2)

In another specific embodiment, the oxyntomodulin derivative of the present invention is a peptide having an amino acid sequence of the following Formula 3, in which a part of the amino acid sequence of oxyntomodulin is linked with the sequence of exendin or GLP-1 using a proper amino acid linker:

R1-B'-C'-R4 (Formula 3)

In still another specific embodiment, the oxyntomodulin derivative of the present invention is a peptide in which a part of the amino acid sequence of oxyntomodulin is substituted with amino acids capable of enhancing the binding affinity to GLP-1 receptor. For example, Leu at position 26, which binds with GLP-1 receptor by hydrophobic interaction, is substituted with the hydrophobic residue, Ile or Val, thereby enhancing the binding affinity to GLP-1 receptor. More specifically, the following Formula 4 is a peptide including the oxyntomodulin peptides having improved receptor binding affinity (D6 substituted):

R1-SQGTFTSDYSKYLD-D1-D2-D3-D4-D5-LFVQW-D6-D7-N-D8-R3 (Formula 4)

In still another specific embodiment, the oxyntomodulin derivative of the present invention is a peptide including the following Formula 5, in which a part of the amino acid sequence is deleted, added, or substituted with another amino acid in order to enhance the activities of native oxyntomodulin on GLP-1 receptor and glucagon receptor:

R1-E1-QGTFTSDYSKYLD-E2-E3-RA-E4-E5-Fv-E6-WLMNT-E7-R5 (Formula 5)

In Formulae 2 to 5, R1 is the same as in the description of Formula 1;
A' is selected from the group consisting of SQGTFTSDYSKYLDSRRAQDFVQWLMNT (SEQ ID NO: 43), SQGTFTSDYSKYLDEEAVRLFIEWLMNT (SEQ ID NO: 44), SQGTFTSDYSKYLDERRAQDFVAWLKNT (SEQ ID NO: 45), GQGTFTSDYSRYLEEEAVRLFIEWLKNG (SEQ ID NO: 46), GQGTFTSDYSRQMEEEAVRLFIEWLKNG (SEQ ID NO: 47), GEGTFTSDLSRQMEEEAVRLFIEWAA (SEQ ID NO: 48), and SQGTFTSDYSRQMEEEAVRLFIEWLMNG (SEQ ID NO: 49);
B' is selected from the group consisting of SQGTFTSDYSKYLDSRRAQDFVQWLMNT (SEQ ID NO: 43), SQGTFTSDYSKYLDEEAVRLFIEWLMNT (SEQ ID NO: 44), SQGTFTSDYSKYLDERRAQDFVAWLKNT (SEQ ID NO: 45), GQGTFTSDYSRYLEEEAVRLFIEWLKNG (SEQ ID NO: 46), GQGTFTSDYSRQMEEEAVRLFIEWLKNG (SEQ ID NO: 47), GEGTFTSDLSRQMEEEAVRLFIEWAA (SEQ ID NO: 48), SQGTFTSDYSRQMEEEAVRLFIEWLMNG (SEQ ID NO: 49), GEGTFTSDLSRQMEEEAVRLFIEW (SEQ ID NO: 50), and SQGTFTSDYSRYLD (SEQ ID NO: 51);
C' is a peptide having 2 to 10 amino acids consisting of combinations of alanine, glycine, and serine;
D1 is serine, glutamic acid, or arginine;
D2 is arginine, glutamic acid, or serine;
D3 is arginine, alanine, or valine;
D4 is arginine, valine, or serine;
D5 is glutamine, arginine, or lysine;
D6 is isoleucine, valine, or serine;
D7 is methionine, arginine, or glutamine;
D8 is threonine, glycine, or alanine;
E1 is serine, AIB, Sar, D-alanine, or D-serine;
E2 is serine or glutamic acid;
E3 is arginine or lysine;
E4 is glutamine or lysine;
E5 is aspartic acid or glutamic acid;
E6 is glutamine, cysteine, or lysine;
E7 is cysteine, lysine, or deleted;
R3 is KRNRNNIA (SEQ ID NO: 37), GPSSGAPPPS (SEQ ID NO: 38), or GPSSGAPPPSK (SEQ ID NO: 39);
R4 is HSQGTFTSDYSKYLD (SEQ ID NO: 40), HSQGTFTSDYSRYLDK (SEQ ID NO: 41), or HGEGTFTSDLSKQMEEEAVK (SEQ ID NO: 42); and
R5 is KRNRNNIA (SEQ ID NO: 37), GPSSGAPPPS (SEQ ID NO: 38), GPSSGAPPPSK (SEQ ID NO: 39), or deleted (excluded if the amino acid sequences of Formulae 2 to 5 are identical to that of SEQ ID NO: 3).

Specifically, the oxyntomodulin derivative of the present invention may be a peptide of the following Formula 6:

R1-X1-X2-GTFTSD-X3-X4-X5-X6-X7-X8-X9-X10-X11-X12-X13- X14-X15-X16-X17-X18-X19-X20-X21-X22-X23-X24-R2 (Formula 6)

wherein R1 is histidine, desamino-histidyl, 4-imidazoacetyl, or tyrosine;
X1 is AIB (aminoisobutyric acid), glycine, serine, or D-serine;
X2 is glutamic acid or glutamine;
X3 is leucine or tyrosine;
X4 is serine or alanine;
X5 is lysine or arginine;
X6 is glutamine or tyrosine;
X7 is leucine or methionine;
X8 is aspartic acid or glutamic acid;
X9 is glutamic acid, alpha-methyl-glutamic acid, or deleted;
X10 is glutamine, glutamic acid, lysine, arginine, or deleted;
X11 is alanine, arginine, or deleted;
X12 is alanine, valine, or deleted;
X13 is lysine, glutamine, arginine, alpha-methyl-glutamic acid, or deleted;
X14 is aspartic acid, glutamic acid, leucine, or deleted;
X15 is phenylalanine or deleted;
X16 is isoleucine, valine, or deleted;
X17 is alanine, cysteine, glutamic acid, glutamine, alpha-methyl-glutamic acid, or deleted;
X18 is tryptophan or deleted;
X19 is alanine, isoleucine, leucine, valine, or deleted;
X20 is alanine, lysine, methionine, arginine, or deleted;
X21 is asparagine or deleted;
X22 is threonine or deleted;
X23 is cysteine, lysine, or deleted;
X24 is a peptide having 2 to 10 amino acids consisting of glycine or is deleted; and R2 is KRNRNNIA (SEQ ID NO: 37), GPSSGAPPPS (SEQ ID NO: 38), GPSSGAPPPSK (SEQ ID NO: 39), HSQGTFTSDYSKYLD (SEQ ID NO: 40), HSQGTFTSDYSRYLDK (SEQ ID NO: 41), HGEGTFTSDLSKQMEEEAVK (SEQ ID NO: 42), or deleted (excluded if the amino acid sequence of Formula 6 is identical to that of SEQ ID NO: 3).

More specifically, the oxyntomodulin derivative of the present invention may be selected from the group consisting of the peptides of SEQ ID NOS: 4 to 36.

Oxyntomodulin has activities of two peptides, GLP-1 and glucagon. GLP-1 decreases blood glucose, reduces food intake, and suppresses gastric emptying, whereas glucagon increases blood glucose, facilitates lipolysis, and decreases body-weight by increasing energy metabolism. If the two peptides having different biological effects are present in one peptide and one of the peptides exhibits a more dominant effect than the other, undesired effects may be generated. If glucagon shows a more dominant effect than GLP-1, blood glucose may be increased. If GLP-1 shows a more dominant effect than glucagon, side effects such as nausea and vomiting may be caused. The overall efficacy may differ depending on the activity ratios of the two peptides.

As used herein, the term "insulinotropic peptide" refers to a peptide possessing an insulinotropic function, and the insulinotropic peptide promotes the synthesis and the expression of insulin in a pancreatic beta cell. The insulinotropic peptide may be exemplified by glucagon-like peptide-1 (GLP-1), glucagon-like peptide-2 (GLP-2), exendin-3, exendin-4, imidazoacetyl (CA) exendin-4, or glucose-dependent insulinotropic peptide (GIP), but is not limited thereto, as long as it possesses the insulinotropic function. The insulinotropic peptide used in the present invention includes all of derivatives, variants, and fragments thereof, in addition to the native insulinotropic peptide. General descriptions thereof are the same as described above.

Descriptions of GLP-1, exendin-3 or exendin-4, and derivatives thereof are included in Korean Patent Publication No. 10-2012-0137271 (or WO2012-169798) regarding the oxyntomodulin derivative and Korean Patent Publication No. 10-2009-0008151 (or WO2009-011544) regarding the insulinotropic peptide derivative, which are incorporated herein by reference, but are not limited thereto.

As used herein, the term "glucagon" refers to a peptide hormone that increases blood glucose levels. The glucagon functions to degrade glycogen into glucose in the liver to increase blood glucose levels.

Meanwhile, the native glucagon may have the following sequence.

His-Ser-Gln-Gly-Thr-Phe-Thr-Ser-Asp-Tyr-Ser-Lys-Tyr-
Leu-Asp-Ser-Arg-Arg-Ala-Gln-Asp-Phe-Val-Gln-Trp-Leu-Met-Asn-
Thr (SEQ ID NO: 52)

Further, the glucagon used in the present invention includes all of derivatives, variants, and fragments thereof, in addition to the native glucagon. General descriptions thereof are the same as described above.

As used herein, the term "parathyroid hormone (PTH)" refers to a hormone released by the parathyroid glands, and acts to increase the concentration of calcium in the blood. Information about an amino acid sequence of the parathyroid hormone may be available from the known database such as GenBank of the US National Institute of Health. The parathyroid hormone includes all of derivatives, variants, and fragments thereof, in addition to the native parathyroid hormone.

As used herein, the term "calcitonin" is a thyroid hormone that regulates the concentration of calcium in the blood, and functions to decrease the concentration of calcium in the blood. Information about an amino acid sequence of the calcitonin may be available from the known database such as GenBank of the US National Institute of Health. The calcitonin includes all of derivatives, variants, and fragments thereof, in addition to the native calcitonin.

The method of the present invention is used to effectively increase solubility of the physiologically active protein or peptide without using a surfactant. However, the surfactant may be used in order to further increase the solubility.

That is, the surfactant is added to the aqueous solution, and the long-acting conjugates of the physiologically active protein or peptide and the immunoglobulin Fc fragment are encapsulated in the micelles, thereby further increasing solubility of the conjugate.

Further, another aspect of the present invention relates to a composition for improving the solubility of the physiologically active protein or peptide, in which the composition includes the immunoglobulin Fc fragment and thus exhibits improved solubility compared to that of a composition containing no immunoglobulin Fc fragment.

Herein, the composition includes the conjugate of the physiologically active protein or peptide and the immunoglobulin Fc fragment in which the physiologically active protein or peptide is linked to the immunoglobulin Fc fragment via the peptide linker or non-peptidyl linker as an active ingredient.

In an embodiment of the present invention, it was examined whether solubility of insulin or oxyntomodulin is increased at a weak acidic pH by conjugating a representative physiologically active peptide, insulin, or oxyntomodulin with the immunoglobulin Fc. As a result, the immunoglobulin Fc fragment-conjugated form significantly increased the solubility of insulin or oxyntomodulin compared to a non-conjugated form (Tables 2 and 3, FIG. 2; and Tables 5 and 6; FIG. 4). This result suggests that the method of the present invention may be used to improve solubility of a peptide or protein, and in particular, to remarkably improve solubility of a peptide or protein which has very low solubility at a neutral or weak acidic condition, and thus a formulation including a pharmaceutically effective concentration thereof is difficult.

Hereinafter, the present invention will be described in more detail with reference to the following Examples. However, these Examples are for illustrative purposes only, and the invention is not intended to be limited by these Examples.

### Example 1: Evaluation of solubility of insulin and long-acting insulin conjugate

### (1) Preparation of long-acting insulin conjugate

Insulin powder was dissolved in 10 mM HCl, and then reacted with 3.4 K propion-ALD₂ PEG (PEG having two propionaldehyde groups, NOF, Japan) at 4°C for about 2 hours at a molar ratio of insulin:PEG of 1:2 and an insulin concentration of 5 mg/mL to PEGylate the N-terminus of the insulin B chain. This reaction was conducted in 50 mM sodium citrate (pH 5.0) and 45% isopropanol, and 2 mM to 20 mM of sodium cyanoborohydride as a reducing agent was added thereto. The reaction solution was purified with an SP HP (GE Healthcare) column using buffer containing sodium citrate (pH 3.0) and a KCl concentration gradient. In order to prepare an insulin-PEG-immunoglobulin Fc fragment conjugate, the mono-PEGylated insulin and immunoglobulin Fc fragment were reacted at a molar ratio of about 1:1.2 with a total protein concentration of 20 mg/mL at room temperature for about 13 hours. In this regard, the reaction solution was 100 mM HEPES and 22 mM potassium phosphate at a pH of 8.2, and 20 mM sodium cyanoborohydride as a reducing agent was added thereto.

After completion of the reaction, the reaction solution was first passed through a Q sepharose HP (GE Healthcare) column using Tris-HCl (pH 7.5) buffer and a NaCl concentration gradient, and then passed through a Source 15 ISO (GE Healthcare) column using Tris-HCl (pH 7.5) buffer and an ammonium sulfate concentration gradient, thereby finally purifying the insulin-PEG-immunoglobulin Fc conjugate.

### (2) Test of solubility of insulin and long-acting insulin conjugate

To examine the solubility of insulin and the long-acting insulin conjugate, a standard and a test material of insulin and the long-acting insulin conjugate were prepared according to the composition as in the following Table 1, respectively.

The test materials of insulin and the long-acting insulin conjugate had a composition of acetic acid buffer solution at a pH of 6.0, polysorbate as a non-ionic surfactant, mannitol as a sugar alcohol, and sodium chloride as an isotonic agent. According to the quantitative values of Tables 2 and 3, the test materials were dissolved at room temperature, the insulin test material that was incompletely dissolved was centrifuged, and only the supernatant was collected and used in the solubility test.

In this regard, as a maximum quantitative value among the theoretical quantitative values of the insulin test material, about 10 mg/mL was determined, which is a quantitative value obtained by converting 100 mg/mL of the long-acting insulin conjugate into insulin. The insulin test material dissolved at the maximum quantitative value was serially diluted, and the point (0.008 mg/mL) at which insulin was completely dissolved without impurities or pellet was determined as a minimum quantitative value.

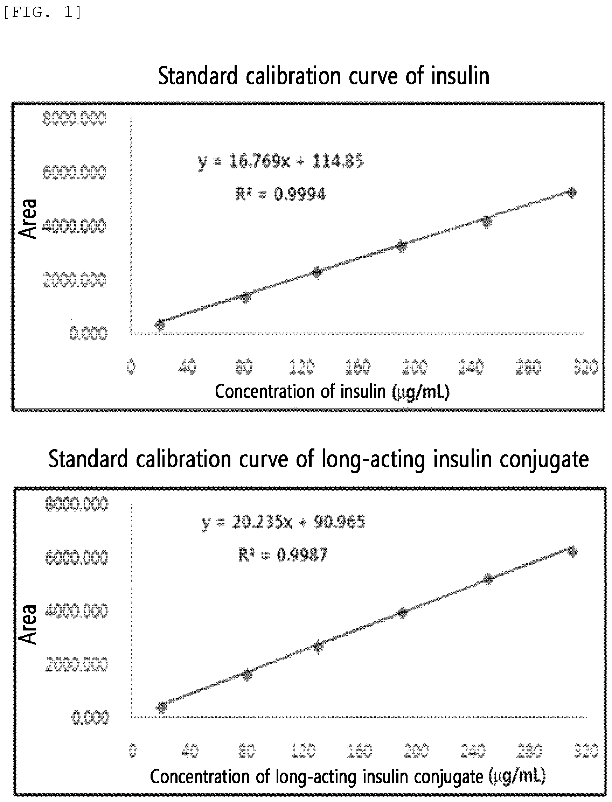

A respective calibration standard curve of insulin and the long-acting insulin conjugate was first confirmed by Reverse Phase-High Performance Liquid Chromatography (RP-HPLC), as in FIG. 1. Quantitative values of insulin and the long-acting insulin conjugate were calculated by putting the peak areas of insulin and the long-acting insulin conjugate test materials confirmed by reverse phase chromatography into the calibration standard curve, respectively. The results are given in Tables 2 and 3, and FIG. 2.

**[Table 1]**

| Material | Dissolution conditions |
|---|---|
| Insulin standard | Dissolved in HCl, and then adjusted to pH 5.0 |
| Insulin test material | pH 6.0 |
| Standard and test material of | pH 6.0 |
| long-acting insulin conjugate | |

**[Table 2]**

| Insulin | |
|---|---|
| Theoretical quantitative value (mg/mL) | Measurement value (quantitative value calculated by standard calibration curve) (mg/mL) |
| 0.008 | 0.000 |
| 0.04 | 0.000 |
| 0.2 | 0.005 |
| 1 | 0.047 |
| 5 | 0.253 |
| 10 | 0.628 |

**[Table 3]**

| Long-acting insulin conjugate (on basis of insulin) | |
|---|---|
| Theoretical quantitative value (mg/mL) | Measurement value (quantitative value calculated by standard calibration curve) (mg/mL) |
| 61.1 | 61.2 |
| 89 | 86.7 |
| 110 | 103.4 |
| 146 | 133.5 |

As shown in Tables 2 and 3, the quantitative value of the long-acting insulin conjugate test material dissolved in the long-acting insulin conjugate formulation buffer showed little difference between the theoretical value and the measurement value, whereas the quantitative value of the insulin test material dissolved in the long-acting insulin conjugate formulation buffer showed a great difference (up to about 16 times) between the theoretical value and the measurement value. Further, when the insulin test material was dissolved in the long-acting insulin conjugate formulation buffer, pellets were observed after centrifugation in all cases, excluding 0.008 mg/mL.

As shown in the result, the solubility was improved by the immunoglobulin Fc fragment used as a carrier of the long-acting insulin conjugate.

### Example 2: Evaluation of solubility of oxyntomodulin and long-acting oxyntomodulin conjugate

### (1) Preparation of long-acting oxyntomodulin derivative conjugate

An oxyntomodulin derivative was reacted with MAL-10 K-ALD PEG (NOF, Japan) at 4°C for about 1 hour at a molar ratio of 1:1 and the oxyntomodulin derivative concentration of 3 mg/mL to PEGylate MAL-10K-ALD PEG at the cysteine residue at position 30 of SEQ ID NO: 27, which is an amino acid sequence of the oxyntomodulin derivative. This reaction was conducted in 50 mM Tris (pH 7.5) and 60% isopropanol. After completion of the reaction, the reaction solution was applied to a SP HP (GE healthcare, Sweden) column using sodium citrate (pH 3.0) buffer and a KCl concentration gradient to purify the oxyntomodulin derivative mono-PEGylated at cysteine.

Next, the thus-purified mono-PEGylated oxyntomodulin derivative and immunoglobulin Fc were reacted at a molar ratio of 1:4 with a total protein level of 20 mg/mL at 4°C for about 16 hours. In this regard, the reaction solution was 100 mM potassium phosphate at a pH of 6.0, and 20 mM sodium SCB as a reducing agent was added thereto. After completion of the reaction, the reaction solution was first passed through a Butyl Sepharose FF (GE Healthcare, Sweden) column using Bis-Tris buffer and a NaCl concentration gradient, and then passed through a Source ISO (GE Healthcare, Sweden) column using sodium citrate buffer and an ammonium sulfate concentration gradient, thereby finally purifying the oxyntomodulin derivative-PEG-immunoglobulin Fc conjugate.

### (2) Test of solubility of oxyntomodulin and long-acting oxyntomodulin conjugate

To examine the solubility of the oxyntomodulin derivative of SEQ ID NO: 27 and the long-acting oxyntomodulin derivative conjugate, a standard and a test material of oxyntomodulin derivative and the long-acting oxyntomodulin derivative conjugate were prepared according to the composition as in the following Table 4, respectively.

The test materials of the oxyntomodulin derivative and the long-acting oxyntomodulin derivative conjugate had a composition of citrate buffer solution at a pH of 5.6, polysorbate as a non-ionic surfactant, mannitol as a sugar alcohol, and methionine. According to the quantitative values of Tables 5 and 6, the test materials were dissolved at room temperature, the oxyntomodulin derivative test material that was incompletely dissolved was centrifuged, and only the supernatant was collected and used in the solubility test.

In this regard, as a maximum quantitative value among the theoretical quantitative values of the oxyntomodulin derivative test material, about 5 mg/mL was determined, which is a quantitative value obtained by converting 80 mg/mL of the long-acting oxyntomodulin derivative conjugate into the oxyntomodulin derivative. The oxyntomodulin derivative test material dissolved at the maximum quantitative value was serially diluted, and the point (0.008 mg/mL) at which insulin was completely dissolved without impurities or pellet was determined as a minimum quantitative value.

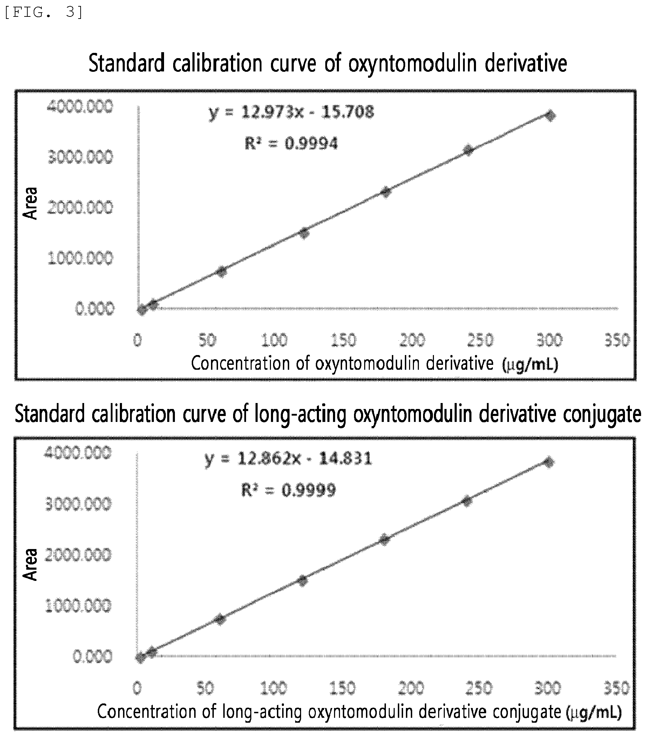

A respective calibration standard curve of oxyntomodulin derivative and the long-acting oxyntomodulin derivative conjugate was first confirmed by reverse phase chromatography, as in FIG. 3. Quantitative values of the oxyntomodulin derivative and the long-acting oxyntomodulin derivative conjugate were calculated by putting the peak areas of the oxyntomodulin derivative and the long-acting oxyntomodulin derivative conjugate test materials confirmed by reverse phase chromatography into the calibration standard curve, respectively.

**[Table 4]**

| Material | Dissolution conditions |
|---|---|
| Oxyntomodulin derivative standard | pH 7.5, 60% isopropanol |
| Oxyntomodulin derivative test material | pH 5.6 |
| Standard and test material of long-acting oxyntomodulin derivative conjugate | pH 5.6 |

**[Table 5]**

| Oxyntomodulin derivative | |
|---|---|
| Theoretical quantitative value (mg/mL) | Measurement value (quantitative value calculated by standard calibration curve) (mg/mL) |
| 0.008 | 0.000 |
| 0.04 | 0.000 |
| 0.2 | 0.002 |
| 1 | 0.014 |
| 5 | 0.188 |

**[Table 6]**

| Long-acting oxyntomodulin derivative conjugate | |
|---|---|
| Theoretical quantitative value (mg/mL) | Measurement value (quantitative value calculated by standard |
| | calibration curve) (mg/mL) |
| 10 | 9.6 |
| 40 | 40.5 |
| 90 | 88.0 |
| 160 | 145.8 |

As shown in Tables 5 and 6, and FIG. 4, the quantitative value of the long-acting oxyntomodulin derivative conjugate test material dissolved in the long-acting oxyntomodulin conjugate formulation buffer showed little difference between the theoretical value and the measurement value, whereas the quantitative value of the oxyntomodulin derivative test material dissolved in the long-acting oxyntomodulin conjugate formulation buffer showed a great difference (up to about 27 times) between the theoretical value and the measurement value. Further, when the oxyntomodulin derivative test material was dissolved in the long-acting oxyntomodulin conjugate formulation buffer, pellets were observed after centrifugation in all cases, excluding 0.008 mg/mL. As shown in the result, the solubility of the oxyntomodulin derivative was improved by the immunoglobulin Fc fragment used as a carrier of the long-acting oxyntomodulin derivative conjugate.

These results indicate that the immunoglobulin Fc fragment used as a carrier of the physiologically active protein and peptide of the present invention is able to effectively improve the solubility of the protein and peptide.

Based on the above description, it will be understood by those skilled in the art that the present invention may be implemented in a different specific form without changing the technical spirit or essential characteristics thereof. Therefore, it should be understood that the above embodiment is not limitative, but illustrative in all aspects. The scope of the invention is defined by the appended claims rather than by the description preceding them, and therefore all changes and modifications that fall within metes and bounds of the claims, or equivalents of such metes and bounds, are therefore intended to be embraced by the claims.

The following numbered clauses, describing aspects of our proposals, are part of the description
[Clause 1] A method for improving the solubility of a physiologically active protein or peptide compared to that of a physiologically active protein or peptide which is not conjugated to an immunoglobulin Fc fragment, wherein the method comprises conjugating the physiologically active protein or peptide to an immunoglobulin Fc fragment.
[Clause 2] The method of clause 1, wherein the immunoglobulin Fc fragment is an Fc fragment derived from IgG, IgA, IgD, IgE, or IgM.
[Clause 3] The method of clause 2, wherein the immunoglobulin Fc fragment is a hybrid of domains in which each domain has a different origin derived from immunoglobulin selected from the group consisting of IgG, IgA, IgD, IgE, and IgM.
[Clause 4] The method of clause 2, wherein the immunoglobulin Fc fragment is a dimer or a multimer consisting of single chain immunoglobulins of the same origin.
[Clause 5] The method of clause 4, wherein the immunoglobulin Fc fragment is a human aglycosylated IgG4 Fc fragment.
[Clause 6] The method of clause 1, wherein the conjugating is to link a physiologically active protein or peptide to an immunoglobulin Fc fragment via a non-peptidyl polymer.
[Clause 7] The method of clause 1, wherein the physiologically active protein or peptide conjugated with the immunoglobulin Fc fragment is in the form of a fusion protein.
[Clause 8] The method of clause 6, wherein the non-peptidyl polymer is polyethylene glycol.
[Clause 9] The method of clause 6, wherein the non-peptidyl polymer is selected from the group consisting of polypropylene glycol, copolymer of ethylene glycol and propylene glycol, polyoxyethylated polyol, polyvinyl alcohol, polysaccharide, dextran, polyvinyl ethyl ether, a biodegradable polymer such as polylactic acid (PLA) and polylactic-glycolic acid (PLGA), a lipid polymer, chitin, hyaluronic acid, and a combination thereof.
[Clause 10] The method of clause 1, wherein the method is for improving the solubility of a physiologically active protein or peptide in an aqueous solution.
[Clause 11] The method of clause 10, wherein the aqueous solution comprises a citric acid or acetic acid buffer solution, polysorbate as a non-ionic surfactant, mannitol as a sugar alcohol, and sodium chloride or methionine as an isotonic agent.
[Clause 12] The method of clause 10, wherein the aqueous solution has a pH from 5.0 to 7.0.
[Clause 13] The method of clause 1, wherein the physiologically active protein or peptide is exendin-4, glucagon, glucagon-like peptide-1 (GLP-1), glucagon-like peptide-2 (GLP-2), parathyroid hormone (PTH), calcitonin, insulin, or oxyntomodulin.
[Clause 14] A composition for improving the solubility of a physiologically active protein or peptide, comprising an immunoglobulin Fc fragment, wherein the composition improves the solubility compared to a composition without an immunoglobulin Fc fragment.
[Clause 15] The composition of clause 14, wherein the composition comprises a conjugate of physiologically active protein or peptide and an immunoglobulin Fc fragment in which a physiologically active protein or peptide is linked to an immunoglobulin Fc fragment via a peptidyl linker or a non-peptidyl linker as an active ingredient.

## Claims

1. A composition for improving the solubility of a physiologically active protein or peptide, comprising an immunoglobulin Fc fragment,
wherein the composition improves the solubility of the physiologically active protein or peptide in an aqueous solution having a pH from 5.0 to 7.0 compared to a composition without an immunoglobulin Fc fragment;
wherein the immunoglobulin Fc fragment is linked to the physiologically active protein or peptide via a non-peptidyl polymer; and
wherein the physiologically active protein or peptide is oxyntomodulin.

2. The composition of claim 1, wherein the composition comprises a conjugate of physiologically active protein or peptide and an immunoglobulin Fc fragment in which a physiologically active protein or peptide is linked to an immunoglobulin Fc fragment via a non-peptidyl linker as an active ingredient.

3. The composition of claim 1, wherein the immunoglobulin Fc fragment is an Fc fragment derived from IgG, IgA, IgD, IgE, or IgM.

4. The composition of claim 1, wherein the immunoglobulin Fc fragment is a hybrid of domains in which each domain has a different origin derived from immunoglobulin selected from the group consisting of IgG, IgA, IgD, IgE, and IgM.

5. The composition of claim 1, wherein the immunoglobulin Fc fragment is a dimer or a multimer consisting of single chain immunoglobulins of the same origin.

6. The composition of claim 1, wherein the immunoglobulin Fc fragment is a human aglycosylated IgG4 Fc fragment.

7. The composition of claim 1, wherein the non-peptidyl polymer is polyethylene glycol.

8. The composition of claim 1, wherein the non-peptidyl polymer is selected from the group consisting of polypropylene glycol, copolymer of ethylene glycol and propylene glycol, polyoxyethylated polyol, polyvinyl alcohol, polysaccharide, dextran, polyvinyl ethyl ether, a biodegradable polymer such as polylactic acid (PLA) and polylactic-glycolic acid (PLGA), a lipid polymer, chitin, hyaluronic acid, and a combination thereof.

9. The composition of claim 1, wherein the aqueous solution comprises a citric acid or acetic acid buffer solution, polysorbate as a non-ionic surfactant, mannitol as a sugar alcohol, and sodium chloride or methionine as an isotonic agent.

10. Use of a pegylated immunoglobulin Fc fragment in a method for improving the solubility of a physiologically active protein or peptide in an aqueous solution having a pH from 5.0 to 7.0 compared to the physiologically active protein or peptide without a pegylated immunoglobulin Fc fragment, the method comprising conjugating the pegylated immunoglobulin Fc fragment to the physiologically active protein or peptide, wherein the physiologically active protein or peptide is oxyntomodulin.
